# EUROPÄISCHE PATENTSCHRIFT

(11) **EP 3 127 563 B1**
(45) Veröffentlichungstag und Bekanntmachung des Hinweises auf die Patenterteilung: **01.03.2023**
(21) Anmeldenummer: 16190456.0
(22) Anmeldetag: 31.10.2014
(51) Int. Cl.: A61M 60/13, A61M 60/148, A61M 60/237, A61M 60/414, A61M 60/808, A61M 60/81, A61M 60/825

(54) **PUMPE, INSBESONDERE BLUTPUMPE**
PUMP, IN PARTICULAR A BLOOD PUMP
POMPE, NOTAMMENT POMPE D'ASSISTANCE CIRCULATOIRE

(30) Priorität: 01.11.2013 EP 13191304
(43) Veröffentlichungstag der Anmeldung: 08.02.2017
(62) Teilanmeldung aus: 14792524.2
(73) Patentinhaber: ECP Entwicklungsgesellschaft mbH, 52074 Aachen (DE)
(72) Erfinder: Scheckel, Mario, 14195 Berlin (DE); Decke, Robert, 12249 Berlin (DE)
(74) Vertreter: Pfenning, Meinig & Partner mbB

(56) Entgegenhaltungen:
- EP-A1- 2 047 872
- WO-A1-2011/003043
- US-A1- 2013 177 409
- US-A1- 2013 204 362

## Beschreibung

Die Erfindung liegt auf dem Gebiet der Mechanik, der Feinwerktechnik sowie der Materialtechnik und bezieht sich auf eine Pumpe bzw. Pumpenanordnung, insbesondere eine Blutpumpe.

Im Stand der Technik sind Pumpen mit einem proximalen und einem distalen Ende sowie einem dazwischen angeordneten Pumpengehäuse, einer in einem Innenraum des Pumpengehäuses entlang einer Längsrichtung angeordneten Antriebswelle, einem auf der Antriebswelle angeordneten Förderelement sowie einer proximal des Pumpengehäuses angeordneten Kanüle bzw. eines Katheters bekannt. Derartige Pumpen weisen oftmals eine flexible Antriebswelle auf, so dass die Pumpen auch an schwer zugängliche Orte geführt werden können und dort ihre Pumpwirkung entfalten. Ein Beispiel ist eine Blutpumpe, welche beispielsweise durch die Femoralarterie über den Aortenbogen in das linke Ventrikel des Herzens eingeschoben wird und im Bereich der Aortenklappe verbleibt. Am proximalen Ende der Pumpe, das heißt beispielsweise, an jenem Ende der Antriebswelle, welches außerhalb des Körpers angeordnet bleibt, kann die Pumpe mit einem Motor verbunden werden, welcher die Antriebswelle antreibt und somit das auf der Antriebswelle angeordnete Förderelement, welches nunmehr beispielsweise im linken Ventrikel angeordnet ist, antreibt. Hierdurch kann Blut aus dem Ventrikel in die Aorta gepumpt werden.

Bei derartigen Pumpen ist es weiterhin bekannt, dass das Pumpengehäuse derart ausgebildet ist, dass es unter Beaufschlagung mit einer zum proximalen Ende der Pumpe wirkenden Kraft zumindest teilweise in die Kanüle bzw. den Katheter überführbar ist. Das heißt, durch das Aufbringen beispielsweise einer Zugkraft im Bereich des proximalen Endes der Antriebswelle kann das Pumpengehäuse in die Kanüle gezogen werden und so von einem expandierten Zustand mit einer größeren Radialausdehnung in einen komprimierten Zustand mit einer kleineren radialen Ausdehnung gebracht werden. Dieses Überführen bietet sich insbesondere vor dem Einbringen bzw. Ausführen der Pumpe in bzw. aus dem Körper an, da der verringerte Durchmesser des Pumpengehäuses die Navigation des distalen Endes der Pumpe innerhalb des menschlichen Körpers erleichtert und insbesondere einen minimal invasiven Hautdurchgang gewährleistet. Das Pumpengehäuse ist hierbei zumeist aus einem Metall, wie beispielsweise einem Memory Shape-Metall, gefertigt. Weitere Werkstoffe können für das Pumpengehäuse verwendet werden, sofern sie den mechanischen Beanspruchungen während der Kompression und der Expansion standhalten sowie den medizinischhygienischen Standards entsprechen.

Weiterhin ist es bei derartigen Pumpen nicht unüblich, dass das Förderelement, wie beispielsweise ein Rotor, mindestens ein faltbares bzw. flexibles Segment, beispielsweise in Form eines Rotorblattes umfasst. Ein Beispiel für einen derartigen Rotor ist beispielsweise in der US 13/261,565 erläutert.

Hinsichtlich des Pumpengehäuses wird beispielsweise auf die US 13/146,452 verwiesen. Weiterhin wird auf die US 13/261,256 verwiesen.

Weiterer relevanter Stand der Technik ist beispielsweise in den Dokumenten EP2047872 A1, US2013/204362 A1, US2013/177409 A1 und WO2011/003043 A1 offenbart.

Bei der Konzeption des Pumpengehäuses hat es sich als eine Möglichkeit erwiesen, einen Abschnitt zu schaffen, welcher im expandierten Zustand des Pumpengehäuses entlang einer entlang der Antriebswelle verlaufenden Längsachse sich um diese Längsachse vom proximalen zum distalen Ende der Pumpe hin betrachtend, spiralartig um die Längsachse windet. Hierbei ist unter einer spiralartig bzw. -förmig verlaufenden Struktur, insbesondere einer spiralartig bzw. -förmig Strebe, jedoch nicht zu verstehen, dass diese vollumfänglich die Längsachse umschließen muss. Es sind hierunter auch Teilabschnitte einer Spirale zu verstehen, welche lediglich ein Teilsegment einer Spirale um die Längsachse bilden, d.h. es kann auch von einer gekrümmten Strebe gesprochen werden, welche im Wesentlichen einem Verlauf einer Spirale um die Längsachse herum über einen Teilabschnitt folgt.

Bei der Entwicklung derartiger Pumpen haben die Erfinder erkannt, dass ein vorteilhaftes Zusammenwirken zwischen dem Pumpengehäuse, der Antriebswelle und dem Förderelement hilfreich ist, um eine effiziente und über einen längeren Zeitraum implantierbare Blutpumpe zu schaffen.

Diese Aufgabe wird mittels einer Pumpe gemäß den Merkmalen des Anspruchs 1 gelöst.

Gemäß einem ersten Aspekt der Erfindung sind die spiralförmig verlaufenden Strukturen oder eine spiralförmig verlaufende Struktur (Einzahl) derart ausgebildet, dass beim Überführen des Pumpengehäuses vom expandierten in den komprimierten Zustand ein entgegen einer ersten Richtung gerichtetes Drehmoment auf das faltbare Segment wirkt. Hierbei sei erwähnt, dass in dieser Anmeldung des Öfteren von einem im Uhrzeigersinn oder gegen den Uhrzeigersinn wirkenden Drehmoment gesprochen wird. Genau genommen wird hierbei nicht auf das Drehmoment referenziert, sondern auf die Richtung der das Drehmoment erzeugenden Kraft verwiesen. Das Drehmoment ist das Vektorprodukt aus dem radialen, von einer Längsachse nach Außen gerichteten Ortsvektor und der erzeugenden Kraft und verläuft somit senkrecht zur erzeugenden Kraft. Wird also von einem im Uhrzeigersinn verlaufenden Drehmoment geschrieben, handelt es sich vielmehr um ein Drehmoment, welches parallel zur Längsachse verläuft. Der Einfachheit und der besseren Orientierung halber wird jedoch oftmals die Richtung des Drehmoments mit der Richtung der erzeugenden Kraft gleichgesetzt, auch wenn dies nicht der physikalischen Definition entspricht.

Der Abschnitt, welcher die spiralförmig verlaufenden Strukturen umfasst, bildet vorzugsweise lediglich einen begrenzten Teilabschnitt des Pumpengehäuses. Dieser Abschnitt führt dazu, dass das Pumpengehäuse beim Einziehen in die Kanüle ein Drehmoment entwickelt, welches entgegen der Richtung der spiralförmigen Windung gerichtet ist. Der Rotor hat aufgrund seiner Form und seiner flexiblen bzw. faltbaren Segmente eine Tendenz, sich beim Komprimieren des Gehäuses in einer bestimmten Richtung um die Antriebswelle zu wickeln. Da das Drehmoment beim Komprimieren des Gehäuses entsteht, kann dies ebenfalls auf das faltbare Segment wirken und somit beispielsweise das faltbare Segment in einer ihm vorbestimmten Faltrichtung mitnehmen.

Dies bedeutet, dass das durch das Pumpengehäuse aufgebrachte Drehmoment die natürliche Faltung des flexiblen Segments um die Antriebswelle herum unterstützt und hierdurch Beschädigungen am Rotor entgegenwirkt.

In einer ersten Ausführungsform des ersten Aspekts ist das faltbare Segment des Förderelements derart ausgebildet, dass das Drehmoment einer Drehrichtung des Förderelements zur Förderung eines Fluids vom distalen zum proximalen Ende der Pumpe hin gleichgerichtet ist. In anderen Worten ist die erste Richtung, in welcher die spiralförmigen Strukturen verlaufen, der Rotation des Förderelements im Betrieb bei einer Förderung des Fluids vom distalen zum proximalen Ende der Pumpe hin entgegengesetzt, wenn die flexiblen Segmente des Förderelements entsprechend ausgebildet sind. Erstaunlicherweise hat sich gezeigt, dass hierdurch sowohl eine Verbesserung des Wirkungsgrads der Pumpe möglich ist, als auch mögliche Beschädigungen des Pumpengehäuses verringert werden können.

In einer weiteren Ausführungsform ist das faltbare Segment des Förderelements derart beschaffen, dass das Drehmoment einer Drehrichtung des Förderelements gleichgerichtet ist und zudem die Entfaltungsrichtung des mindestens einen faltbaren Segments beim Entfalten in der ersten Richtung verläuft. Das heißt, die Drehrichtung des Förderelements beim Fördern des Fluids vom distalen zum proximalen Ende hin ist der Entfaltungsrichtung des Rotors entgegengesetzt.

In einer weiteren Variante kann das faltbare Segment des Förderelements derart ausgebildet sein, dass das Drehmoment einer Drehrichtung des Förderelements zur Förderung eines Fluids vom distalen zum proximalen Ende der Pumpe entgegengesetzt ist.

In einer weiteren Ausführungsform ist die Entfaltungsrichtung des mindestens einen faltbaren Segments beim Entfalten in einer der ersten Richtung entgegengesetzten Richtung.

In einer weiteren Ausführungsform ist das Pumpengehäuse aus einem Memory Shape-Material hergestellt. Hierbei kann das Pumpengehäuse beispielsweise aus Nitinol gefertigt sein.

In einer weiteren Ausführungsform ist es vorgesehen, wenn die "austentite finish" (A_{f})-Temperatur des Pumpengehäuses unterhalb der Körpertemperatur eines Menschen im gesunden Zustand, insbesondere unter 30°C und insbesondere unter der Raumtemperatur, d.h. unter 20°C liegt. Überraschenderweise hat sich gezeigt, dass bei dieser A_{f}-Temperatur die Stabilität und die Langlebigkeit des Gehäuses verbessert werden kann. Dies gilt insbesondere dann, wenn die A_{f}-Temperatur unterhalb der Raumtemperatur liegt.

In einer weiteren Ausführungsform umfasst das Pumpengehäuse einen pumpenaufnehmenden und einen proximal des pumpenaufnehmenden Abschnitts angeordneten proximalen Abschnitt, wobei ein Innendurchmesser des proximalen Abschnitts von einem Durchmesser des pumpenaufnehmenden Abschnitts im expandierten Zustand des Pumpengehäuses zu einem proximalen Ende des proximalen Abschnitts hin verringert ist. Mittels eines derartigen Pumpengehäuses wird das Einziehen in die Kanüle vereinfacht und aufgrund der Form des Pumpengehäuses unterstützt. Bei der erfindungsgemäßen Pumpe umfassen die spiralförmigen Strukturen spiralförmige Streben, die im proximalen Abschnitt angeordnet sind.

In einer weiteren Ausführungsform sind die spiralförmigen Strukturen sowohl im proximalen als auch im pumpenaufnehmenden Abschnitt angeordnet.

In einer weiteren Ausführungsform umfasst das Pumpengehäuse einen weiteren, distal des pumpenaufnehmenden Abschnitts angeordneten distalen Abschnitt, dessen Innendurchmesser vorzugsweise von einem Durchmesser des pumpenaufnehmenden Abschnitts im expandierten Zustand des Pumpengehäuses zu einem distalen Ende des distalen Abschnitts hin verringert ist.

Hierdurch ist ein verbesserter Schutz des Rotors möglich, da die Antriebswelle beispielsweise im Bereich des verringerten Innendurchmessers des distalen Abschnitts durch ein weiteres Lager gelagert sein kann.

In einer weiteren Ausführungsform sind die spiralförmigen Strukturen ebenfalls im distalen Abschnitt angeordnet. Dabei können die spiralförmigen Strukturen entgegen der ersten Richtung gewickelt bzw. gewunden sein. In dieser Ausführungsform unterstützen die spiralförmigen Strukturen sowohl im proximalen, als auch im distalen Abschnitt die Bildung eines Drehmoments, welches zwar über das gesamte Pumpengehäuse zwischen dem proximalen und dem distalen Abschnitt hinweg eingeleitet wird, das Drehmoment jedoch lediglich im Bereich des proximalen und distalen Abschnitts eine Verbiegung bzw. Verdrillung der spiralförmigen Elemente bewirkt. In einer Variante sind die spiralförmigen Strukturen im proximalen und distalen Bereich derart ausgebildet, dass das Drehmoment proximal und distal gleichgerichtet ist und/oder das proximale und distale Drehmoment im Wesentlichen gleich groß ist. Dies ist in einer Analogie mit der Wickelung eines Bonbons in einem Bonbonpapier vergleichbar, wobei durch Festhalten und gleichzeitiges Ziehen an beiden Enden ein Entwickeln des Bonbons aus dem Papier möglich ist. Hierdurch wird beispielsweise ein Verdrillen der Antriebswelle verhindert und die Antriebswelle vor Schäden geschützt.

In einer weiteren Ausführungsform ist die Antriebswelle alternativ oder zusätzlich in einem Bereich eines proximalen Endes des Pumpengehäuses gelagert.

In einem zweiten Aspekt der Erfindung ist eine Entfaltrichtung des mindestens einen flexiblen Elements bei einem Überführen des Pumpengehäuses vom komprimierten in den expandierten Zustand hin entgegen der Drehrichtung des Förderelements bei der Förderung eines Fluids vom distalen zum proximalen Ende der Pumpe hin ausgebildet unabhängig von spiralförmigen Strukturen. In diesem Fall, ebenso wie im ersten Aspekt der Erfindung, ist unter der Entfaltrichtung die Bewegung der radial betrachtet äußeren Ende des Segments des Förderelements zu verstehen.

Einen weiterer Aspekt der Erfindung umfasst ein Pumpengehäuse, ein in einem Innenraum des Pumpengehäuses entlang einer Längsachse angeordnete Antriebswelle, sowie ein auf der Antriebswelle angeordnetes Förderelement. Das Pumpengehäuse umfasst zumindest einen pumpenaufnehmenden Abschnitt und ein proximal des pumpenaufnehmenden Abschnitts angeordneten proximalen Abschnitt, wobei das Pumpengehäuse in einer quer zur Längsrichtung verlaufenden Radialrichtung von einem komprimierten Zustand in einen expandierten Zustand überführbar ist. Die Antriebswelle ist im Bereich des proximalen Endes des Pumpengehäuses in einem proximalen Lager gelagert.

In diesem dritten Aspekt der Erfindung ist die Antriebswelle derart konfiguriert, dass eine Biegesteifigkeit der Antriebswelle im Bereich des proximalen Abschnitts des Pumpengehäuses und distal des proximalen Lagers mit einer Biegesteifigkeit des proximalen Abschnitts des Pumpengehäuses korrespondiert. Auf diese Weise sind bei einer Biegung das Pumpengehäuse und das Förderelement im Wesentlichen konzentrisch zueinander innerhalb des pumpenaufnehmenden Abschnitts gelagert. In anderen Worten wird die Biegelinie des Pumpengehäuses im proximalen Abschnitt mit der Biegelinie der flexiblen Welle im Bereich des proximalen Abschnitts harmonisiert, so dass ein auf das distale Ende des Pumpengehäuses wirkendes Biegemoment eine ähnliche Biegung sowohl im Gehäuse als auch in der Welle induziert. Hierdurch wird vermieden, dass der Rotor aufgrund unterschiedlicher Biegesteifigkeiten mit dem Pumpengehäuse kollidiert und das Pumpengehäuse oder den Rotor selbst zerstören kann. Während des Betriebs der Pumpe kann es durch die Bewegung des schlagenden Herzens oder des Patienten zu Biegemomenten bzw. -kräften kommen, welche ohne eine Abstimmung der Biegesteifigkeiten bzw. -momente zu einer Beschädigung von Rotor oder Pumpengehäuse führen können.

In einer Variante ist die Biegesteifigkeit des proximalen Abschnitts des Pumpengehäuses weicher gegenüber dem pumpenaufnehmenden Abschnitt. Im Bereich des proximalen Abschnitts ist auch die flexible Welle weicher gegenüber einem Wellenabschnitt im pumpenaufnehmenden Abschnitt des Gehäuses.

Die Biegesteifigkeit des Pumpengehäuses im proximalen Abschnitt kann beispielsweise durch spiralförmige Strukturen beeinflusst werden. Durch die spiralförmigen Strukturen wird in einem Ausführungsbeispiel ein elastischer Bereich geschaffen, welcher die mechanischen Wechsellasten aufgrund verschieden wirkender Biegemomente abfängt. Dabei ist es in einer Variante vorgesehen, die spiralförmigen Strukturen symmetrisch um die Längsachse herum anzuordnen. Auf diese Weise bilden die spiralförmigen Strukturen einen spiralförmigen Bereich, welcher eine Federwirkung aufweist. Diese Federwirkung erlaubt die Steuerung der gewünschten Biegesteifigkeit. Insbesondere kann über den Winkel bzw. den Spiralverlauf der spiralförmigen Strukturen die gewünschte Biegesteifigkeit eingestellt werden. Um eine Dauerfestigkeit des Pumpengehäuses zu gewährleisten, ist in einer Variante eine maximale lokale Verzerrung an jedem Punkt des Pumpengehäuses kleiner als 2%.

In einer weiteren Ausführungsform umfasst das Pumpengehäuse weiterhin einen distal des pumpenaufnehmenden Abschnitts distalen Abschnitt, wobei die Antriebswelle im Bereich des distalen Endes des Pumpengehäuses in einem distalen Lager gelagert ist und eine Biegesteifigkeit der Antriebswelle im Bereich des distalen Abschnitts und proximal des distalen Abschnitts derart auf eine Biegesteifigkeit des distalen Abschnitts abgestimmt ist, so dass bei einer Biegung des Pumpengehäuses das Förderelement im Wesentlichen konzentrisch innerhalb des pumpenaufnehmenden Abschnitts angeordnet ist. Hierbei kann beispielsweise die Antriebswelle zudem im Bereich des distalen Endes der Pumpe gelagert sein, so dass die Antriebswelle zwischen einem proximalen und einem distalen Lager eingespannt ist. Indem die Antriebswelle im Bereich des distalen und proximalen Abschnitts des Pumpengehäuses eine Biegesteifigkeit aufweist, welche mit einer Biegesteifigkeit des Pumpengehäuses im proximalen bzw. distalen Abschnitt korrespondiert, ist es möglich die im Wesentlichen konzentrische Lagerung des Rotors im Pumpengehäuse zu gewährleisten.

In einer weiteren Ausführungsform ist das Pumpengehäuse derart ausgebildet, dass es mit einer Steifigkeit eines Katheters, beispielsweise im distalen oder proximalen Ende des Pumpenbereichs, abgestimmt ist. Ist der Katheter zu steif, werden starke Verformungen in das Pumpengehäuse eingeleitet, ist dieser jedoch zu weich, ist die Position des Gehäuses im Betrieb nicht sichergestellt, so dass in beiden Fällen der sichere Betrieb des Rotors im Pumpengehäuse nicht gewährleistet werden kann. Durch Abstimmen der Steifigkeit des Pumpengehäuses mit der Steifigkeit des Katheters, wird hier die konzentrische Lagerung des Rotors im pumpenaufnehmenden Abschnitt auch im Betrieb der Pumpe gewährleistet.

Um die Biegesteifigkeit der Welle zu beeinflussen, kann unter anderem vorgesehen sein, eine Hohlwelle zu verwenden, welche im Bereich des pumpenaufnehmenden Abschnitts mit einer Seele versehen ist. Zudem kann sich die Seele bis zum distalen Lager und zum proximalen Lager erstrecken.

Bei den in dieser Anmeldung beschriebenen Pumpenanordnungen wirken in der Realität verschiedene äußere Krafteinwirkungen und Biegewechselbelastungen auf die Antriebswelle, das Pumpengehäuse, eines distal des Pumpengehäuses angeordneten Pigtails und ggf. auf Lagerelemente des Katheters bzw. der Blutpumpenanordnung ein. Äußere Krafteinwirkungen und Biegewechselbelastungen können auf den Katheter übertragen werden beispielsweise durch eine Innenwand des Herzens, an der das Katheter ggf. anliegt bzw. abgestützt wird (bspw. über eine sogenannte Pigtail-Spitze), durch pulsatile Druckänderungen bzw. Strömungsänderungen des Bluts innerhalb einer Herzkammer bzw. eines Blutgefäßes, wie beispielsweise dem linken oder rechten Ventrikel oder der Aorta, durch eine Lage- bzw. Haltungsänderung des Körpers, insbesondere durch eine Rumpfbewegung oder eine (Bein-)Bewegung in der Nähe einer Punktionsstelle. Trotz dieser Belastungen kann mit dem vorgeschlagenen Katheter und der vorgeschlagene Blutpumpenanordnung Blut über längere Zeiträume, wie beispielsweise über Stunden, Tage oder gar Wochen auch bei hohen Drehzahlen des Pumpenrotors, beispielsweise im oben genannten Drehzahlbereich, gefördert werden, wie beispielsweise in der oben beschriebenen Verwendung der Blutpumpenanordnung.

Es wird darauf hingewiesen, dass die in den Unteransprüchen zum unabhängigen Anspruch 1 aufgeführten Merkmalen auch mit den zweiten und dritten Aspekt der Erfindung kombinierbar sind.

Weitere Aspekte werden anhand der nachfolgenden Figuren erläutert.

Es zeigen:
- FIG. 1: eine schematische Übersicht über eine Pumpenanordnung;
- FIG. 2a bis 2d: eine Variante eines Pumpengehäuses mit darin angeordnetem Förderelement auf einer Antriebswelle, welche lediglich proximal gelagert ist;
- FIG. 3a bis 3d: Variante einer Pumpe mit einem Pumpengehäuse und einem auf einer Antriebswelle gelagerten Förderelement, wobei die Antriebswelle distal und proximal gelagert ist;
- FIG. 4a, 4b: Ausführungsbeispiel einer korrespondierenden Biegesteifigkeit zwischen Pumpengehäuse und Antriebswelle;
- FIG. 5a, 5b: weitere Ausführungsformen eines Pumpengehäuses und einer Antriebswelle mit korrespondierenden Biegesteifigkeiten;
- FIG. 6a, 6b: Ausführungsformen einer Antriebswelle mit Seele und Rotor;
- FIG. 7a bis 7c: Ausführungsformen eines Pumpengehäuses;
- FIG. 8: Ausführungsform eines distalen Endes des Pumpengehäuses mit darin anschließendem Katheter;
- FIG. 9: Darstellung einer Pumpenanordnung mit einer abgestimmten Kombination von Pumpengehäuse und Antriebswelle zur Harmonisierung der Biegelinie.

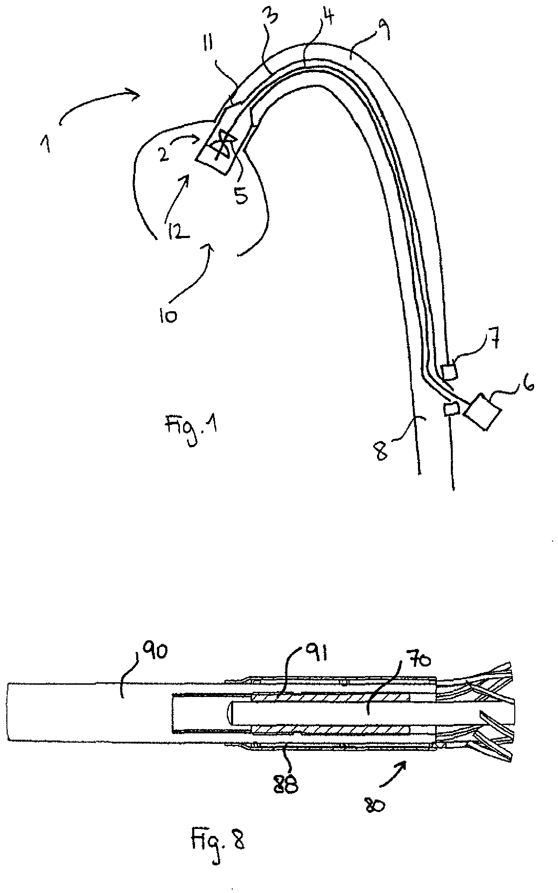

Anhand der FIG. 1 soll eine schematische Übersicht über eine Pumpenanordnung 1 gegeben werden. Die Pumpenanordnung 1 umfasst ein Pumpengehäuse 2 mit einer Kanüle bzw. einem Katheter 3, in welchem eine Antriebswelle 4 angeordnet ist. Im Bereich des Pumpengehäuses 2 befindet sich ein Förderelement 5, welches über die Antriebswelle 4 mit einem am proximalen Ende der Antriebswelle angeschlossenen Motor 6 angetrieben wird. Die Pumpe inkl. der Antriebswelle 4 wird dabei über eine Schleuse 7 beispielsweise durch die Femoralarterie 8 und den Aortenbogen 9 in ein Herzventrikel 10 eingeführt, so dass das Pumpengehäuse 2 im Bereich der Aortenklappe zu liegen kommt. Der Rotor 5 ist dabei derart ausgebildet, dass Blut in Richtung 12 vom Ventrikel in die Aorta, das heißt, vom distalen Ende der Pumpe zum proximalen Ende der Pumpe hin gefördert wird.

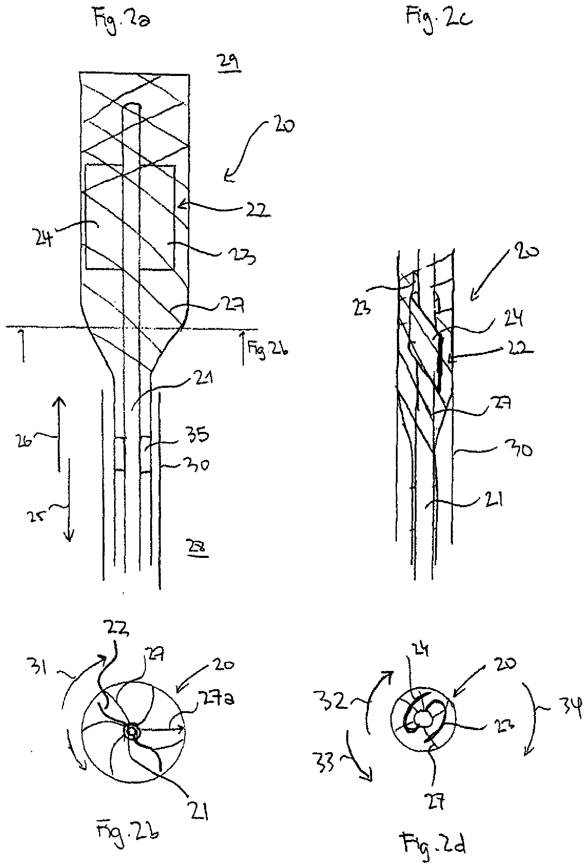

Anhand der FIGN. 2a bis d werden verschiedene Interaktionen zwischen dem Gehäuse, der Antriebswelle, dem Förderelement und der Kanüle erläutert. In den FIG. 2a und 2c ist das Pumpengehäuse 20 im Längsschnitt im expandierten Zustand (FIG. 2a) und dem komprimierten Zustand (FIG. 2c) dargestellt. In den FIG. 2b und 2d finden sich entsprechende Querschnitte.

Im Pumpengehäuse 20 ist die Antriebswelle 21 mit dem darauf befindlichen Förderelement 22 angeordnet. Das Förderelement umfasst im vorliegenden Beispiel zwei flexible Segmente 23 und 24, welche als Rotorblätter ausgeführt sind. Das Überführen des Pumpengehäuses 20 vom expandierten Zustand in den komprimierten Zustand erfolgt durch Ziehen der Antriebswelle in der Zugrichtung 25, welche parallel zur Längsrichtung 26 des Pumpengehäuses ist. In der FIG. 2b ist ein Querschnitt zur Darstellung des Pumpengehäuses in der Darstellung der FIG. 2a dargestellt. Es ist erkennbar, dass das Pumpengehäuse 20 im Wesentlichen konzentrisch um die Antriebswelle 21 herum angeordnet ist. In dem hier dargestellten Schnitt sind die spiralförmig verlaufenden Streben 27 als spiralförmige Strukturen erkennbar, welche sich vom proximalen zum distalen Abschnitt hin in der Radialrichtung 27a aufweiten. Die Streben verlaufen vom proximalen Ende des Pumpengehäuses 28 zum distalen Ende des Pumpengehäuses 29 hin, gegen den Uhrzeigersinn. Im Vergleich dazu ist auch das Förderelement 22 eingezeichnet, dessen flexible Segmente eine Förderung des Fluids bewirken Dies ist auch in der Aufsicht der FIG. 2a erkennbar. Alternativ zur Vielzahl der Streben kann auch eine andere spiralförmige Struktur gewählt werden, wie beispielsweise ein Vielzahl von Streben, welche aufgrund ihrer Anordnung eine spiralförmige Linie, d.h. Struktur, bilden.

Wird nun das Pumpengehäuse 20 vom in der FIG. 2a dargestellten expandierten Zustand durch Ziehen in Zugrichtung 25 in den komprimierten Zustand in die Kanüle 30 eingezogen, bewirken die spiralförmigen Elemente ein Drehmoment 31, welches im Uhrzeigersinn wirkt. Es ist somit im Verlauf der spiralförmigen Streben entgegengesetzt und versucht der Verdrillung der spiralförmigen Streben entgegenzuwirken, auch wenn keine sichtbare Veränderung des Gehäuses erkennbar ist. Durch das Drehmoment wird auf die Segmente 23 und 24 gewirkt, so dass sich die Segmente 23 und 24, wie in der FIG. 2d dargestellt, in der Faltrichtung 32 mit dem Drehmoment 31 um die Antriebswelle 21 einwickeln. Entsprechend entfaltet sich der Rotor beim Hinausschieben des Pumpengehäuses aus der Kanüle 30 in Längsrichtung in der Entfaltrichtung 33.

In dem hier dargestellten Beispiel ist die anschließende Drehrichtung des Rotors in der Drehrichtung 34, welche der Entfaltrichtung entgegengesetzt ist. Hierdurch kann unter anderem ein weiteres Entfalten des Rotors bei höheren Drehzahlen bewirkt werden. In anderen nicht beanspruchten Varianten ist es jedoch möglich, die Drehrichtung in Gleichlauf mit der Entfaltrichtung zu wählen. Hierbei bewirkt eine höhere Drehzahl ein leichtes Einfalten des Rotors in der Faltrichtung 32.

Im vorliegenden Beispiel ist die Antriebswelle aus einer Nickel-KobaltLegierung, wie beispielsweise 35NL T^{®} oder MP35N^{®}. Die Kanüle ist beispielsweise aus einem Katheter aus einem aus dem Stand der Technik bekannten Werkstoff wie beispielsweise Silikon oder Polyurethan. Das Pumpengehäuse kann beispielsweise aus Nitinol gefertigt sein. Dabei ist im vorliegenden Beispiel die A_{f}-Temperatur des Pumpengehäuses bei ca. 15°, so dass die A_{f}-Temperatur unterhalb der Raumtemperatur liegt. Dies hat Vorteile bei der Stabilität des Pumpengehäuses. In dem folgenden Beispiel ist die Antriebswelle lediglich durch eine proximale Lagerhülse 35 gelagert. Hinsichtlich der verwendeten Wertstoffe für den Rotor können beispielsweise die in der US 13/261 565 beschriebenen Werkstoffe eingesetzt werden.

In dem in den FIGN. 2 dargestellten Beispiel verlaufen die spiralförmig angeordneten Streben sowohl im proximalen Abschnitt des Pumpengehäuses, welches proximal des Förderelements 22 liegt, als auch im Bereich des pumpenaufnehmenden Abschnitts, welcher im Bereich des Förderelements 22 liegt.

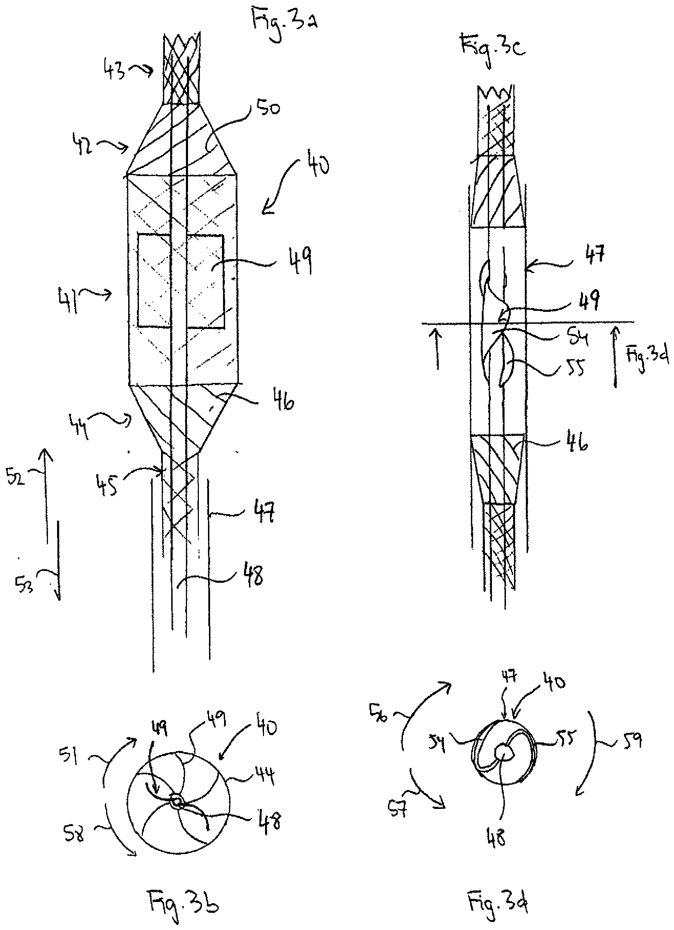

Eine Variante einer Kombination von Pumpengehäuse, Förderelement und Antriebswelle ist beispielsweise in den FIG. 3a bis 3d gezeigt.

Ein Unterschied zwischen den Ausführungsformen der FIG. 2 und der FIG. 3 ist unter anderem, dass die Antriebswelle bei der Ausführungsform der FIG. 3 sowohl in einem distalen Ende als auch im Bereich eines proximalen Endes des Pumpengehäuses gelagert ist.

Das in der FIG. 3a dargestellte Pumpengehäuse 40 umfasst einen pumpenaufnehmenden Abschnitt 41, einen distal des pumpenaufnehmenden Abschnitts angeordneten Abschnitt 42 und einen distal des distalen Abschnitts angeordneten distalen Endabschnitt 43. Weiterhin umfasst das Pumpengehäuse einen proximal des pumpenaufnehmenden Abschnitts angeordneten proximalen Abschnitt 44 und einen proximal des proximalen Abschnitts angeordneten Endabschnitt 45. Das Pumpengehäuse 40 weist im proximalen Abschnitt 44 und dem distalen Abschnitt 42 spiralförmige Streben 46 auf, die beispielsweise in der FIG. 3b gezeigt sind. Die Streben verlaufen dabei vom proximalen Ende der Pumpe zum distalen Ende der Pumpe hin, gegen den Uhrzeigersinn. In der FIG. 3a ist zudem eine Kanüle 47 gezeigt, welcher die Antriebswelle 48 bei ihrem Lauf durch den Aortenbogen und die körpereigenen Gefäße umhüllt. Im Bereich des pumpenaufnehmenden Abschnitts 41 des Gehäuses ist auf der Antriebswelle zudem ein Rotor 49 angeordnet, welcher zur Förderung des Blutes vom distalen zum proximalen Ender hin dient. Anhand der FIG. 3b ist erkennbar, dass die spiralförmigen Streben 46 im proximalen Abschnitt 44 gegen den Uhrzeigersinn und von Innen (d.h. dem distalen Ende des Endabschnitts 45) nach Außen (d.h. zum proximalen Ende des Abschnitts 41) verlaufen, wohingegen die spiralförmigen Streben 50 im distalen Abschnitt 42 mit dem Uhrzeigersinn und von Außen nach Innen verlaufen. Dies hat zur Folge, dass bei Festhalten des distalen Endabschnitts 43 und des proximalen Endabschnitts 45 und Ziehen an beiden Abschnitten in entgegensetzten Richtungen, ein in Richtung des Uhrzeigersinns verlaufendes Drehmoment auf den pumpenaufnehmenden Abschnitt 41 wirkt. Dieser Mechanismus wirkt auch beim Einziehen des Pumpengehäuses in die Kanüle. Korrespondierend zur FIG. 2a ist in der FIG 3a die Pumpe im expandierten Zustand dargestellt. Wirkt nun eine entgegen der Längsrichtung 52 gerichtete Zugkraft 53, wird zum einen der Durchmesser des Pumpengehäuses in den Abschnitten 41, 42 und 44 verringert und zugleich das im Uhrzeigersinn wirkende Drehmoment 51 induziert. Aufgrund der Verringerung des Durchmessers beim Kollabieren interagiert das Pumpengehäuse 40 mit dem Förderelement 49 bzw. dessen flexiblen Segmenten 54 und 55. Die flexiblen Segmente 54 und 55 weisen aufgrund ihrer Form und ihrer Ausrichtung eine Faltrichtung 56 in Richtung des Drehmomentes auf. Auf diese Weise werden die flexiblen Segmente 54 und 55 in der Faltrichtung 56 um die Antriebswelle 48 gewickelt. Wird das Pumpengehäuse nun von der komprimierten Konfiguration in der FIG. 3c in die expandierte Konfiguration der FIG. 3a überführt, entfaltet sich der Rotor in der Entfaltrichtung 57, welche mit der Spiralrichtung 58 der spiralförmigen Streben übereinstimmt. In dem vorliegenden Beispiel dreht der Rotor 48 anschließend in der Richtung 59, um das Blut vom distalen zum proximalen Ende der Pumpe zu führen.

Die in den FIG. 3 gezeigte Ausführungsform entspricht einer "Bonbonwicklung", da die den Verlauf der spiralförmigen Streben definierenden Spiralen im distalen und im proximalen Abschnitt in entgegensetzten Richtungen drehen. Hierdurch wird lediglich ein Drehmoment im pumpenaufnehmenden Abschnitt 41 sowohl als auch dem distalen und proximalen Abschnitt 42 und 44 eingebracht, das in die distalen und proximalen Endabschnitte 43 und 45 wirkende Drehmoment jedoch verringert. Da sich in den distalen und proximalen Endabschnitten Lager (nicht dargestellt) für die Antriebswelle 48 befinden, wird das Drehmoment des Pumpengehäuses ggf. auf die Antriebswelle übertragen, wenn das Pumpengehäuse 40 vom expandierten in den komprimierten Zustand überführt wird.

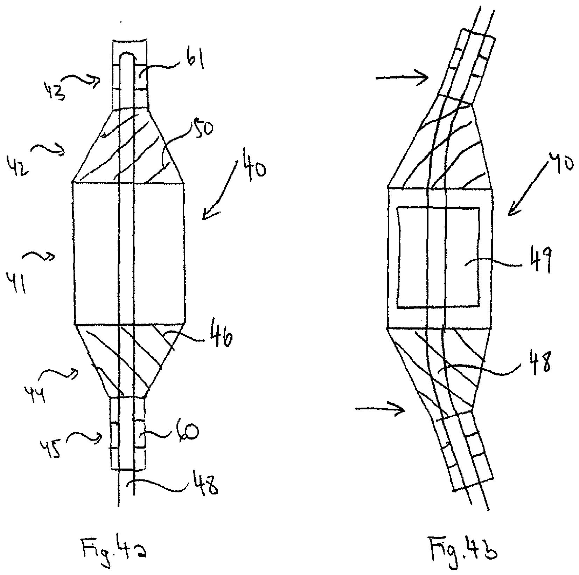

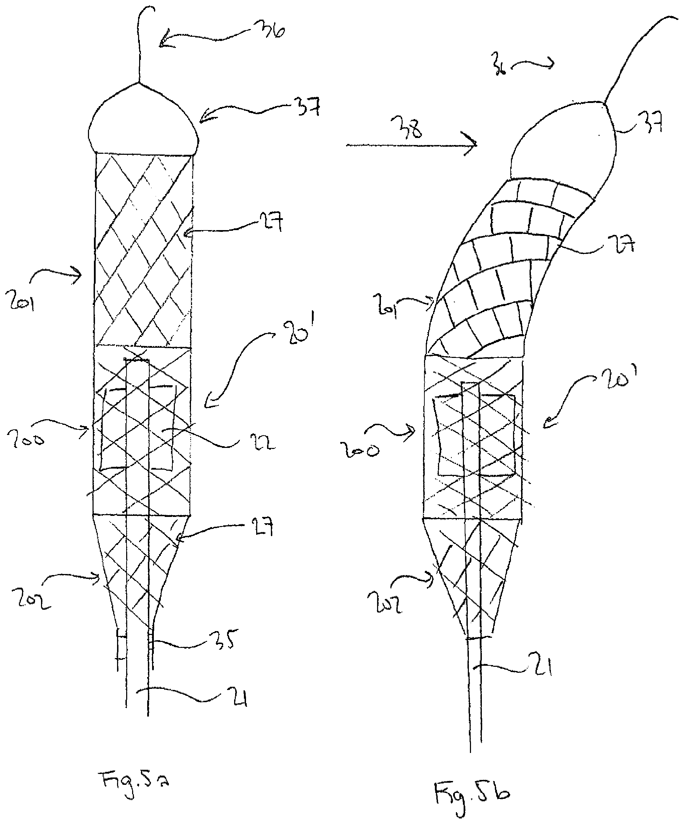

Anhand der FIG. 4a und 4b, sowie der FIG. 5a und 5b soll auf die Aspekte der korrespondierenden Biegesteifigkeit des Pumpengehäuses und der Welle eingegangen werden.

In den FIG. 4a und 4b ist eine der den FIG. 3 entsprechende Pumpenanordnung dargestellt. Insbesondere umfasst das Pumpengehäuse 40 die zu den FIG. 3 beschriebenen Abschnitte 41 bis 45, sowie eine Antriebswelle 48, welche proximal in einem ersten Lager 60 und einem distalen Lager 61 gehalten ist. In den distalen und proximalen Abschnitten 42 bzw. 44 befinden sich spiralförmige Streben 46 bzw. 50. Wird nun ein Biegemoment auf das Pumpengehäuse 40 aufgebracht, wie in der FIG. 4b dargestellt, bewirken die spiralförmigen Streben 46 bzw. 50 unter anderem aufgrund ihrer um die Antriebswelle angeordneten symmetrischen Anordnung eine Biegung des Pumpengehäuses, welche mit einer entsprechenden Biegung der Antriebswelle im distalen bzw. proximalen Abschnitt 42 bzw. 44 korrespondiert. Hierbei kann beispielsweise die Welle in den vorgenannten Bereichen weicher sein, als im Bereich des pumpenaufnehmenden Abschnitts 41. Die Versteifung im pumpenaufnehmenden Abschnitt wird zudem durch den Rotor selbst bzw. die Rotornabe verstärkt. Hierdurch wird bewirkt, dass, wie in der FIG. 4b zu sehen, das Förderelement 49 im Wesentlichen auch bei Biegebelastungen konzentrisch innerhalb des pumpenaufnehmenden Abschnitts verbleibt. Beispielsweise anhand der Dicke der Streben, des gewählten Winkels der spiralförmigen Streben, sowie der Anzahl und Anordnung der Streben kann ein entsprechendes Biegemoment an die Biegesteifigkeit der Welle in dem korrespondierenden Bereich angepasst werden. Das Biegemoment ist hier die Summe des Produkts aus der erzeugenden Kraft und dem entsprechenden Kraftarm über alle angreifenden Kräfte. Der Kraftarm ist dabei der Abstand von einem Auflagepunkt. Als Auflagepunkt kann beispielsweise ein Punkt im Bereich des proximalen Lagers gewählt werden.

In den FIG. 5a und 5b ist ein korrespondierender Fall dargestellt, wobei die Pumpenanordnung hier im Wesentlichen der Pumpenanordnung der FIGN. 2 entspricht. Allerdings weist das Pumpengehäuse 20' in diesem Fall einen steifen, pumpenaufnehmenden Abschnitt und einen distal eines pumpenaufnehmenden Abschnitts 200 gelegenen distalen Abschnitt 201 auf, welcher spiralförmige Strukturen 27 aufweist. Die spiralförmigen Strukturen 27, welche aufgrund einer leiterartigen Anordnung einer Vielzahl von Streben und deren Verbindungen oder durch segmentweises Rotieren von Strebenstrukturen erzeugt werden können, sind derart konfiguriert, dass die Biegesteifigkeit des Pumpengehäuses im distalen Abschnitt 201 weicher ist als im pumpenaufnehmenden Abschnitt 202. Hierdurch können Biegemomente, welche auf den Pigtail 36 wirken, neben der distalen Übergangsstruktur 37, welche beispielsweise aus 4 Streben aufgebaut sein kann, auch durch den distalen Abschnitt aufgenommen werden. Auf diese Weise wirkt das Biegemoment 38 (Fig. 5b) nicht auf den pumpenaufnehmenden Abschnitt, so dass die Antriebswelle auch beim Anliegen von Biegemomenten im Wesentlichen konzentrisch innerhalb des pumpenaufnehmenden Abschnitts liegt. Der pumpenaufnehmende Abschnitt 200 ist steifer, wobei Maßnahmen zur Erhöhung der Biegesteifigkeit in einem der späteren Ausführungsbeispiele erläutert werden.

Optional kann das Pumpengehäuse auch einen proximalen Abschnitt 202 mit spiralförmigen Strukturen 27 umfassen, um ein wirkendes Biegemoment zu kompensieren und um die Komprimierung des Pumpengehäuses zu vereinfachen.

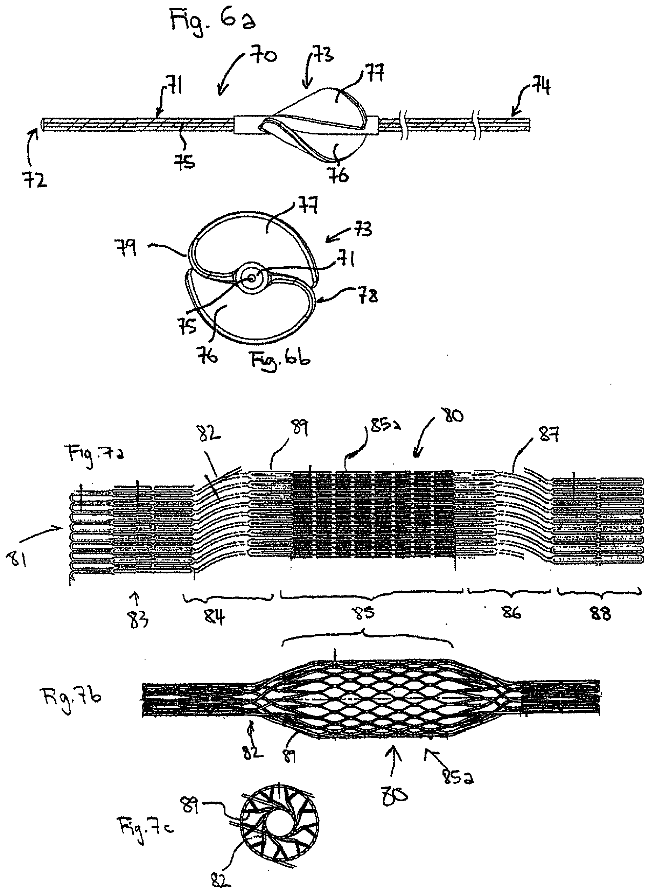

Anhand der FIG. 6a und 6b soll auf weitere Details der verschiedenen Aspekte der Erfindung eingegangen werden. Die Wellenanordnung 70 umfasst die Antriebswelle 71 mit einem distalen Ende 72, einem Förderelement 73, sowie einem proximalen Ende 74, welches beispielsweise mit einem Kopplungselement mit einem Motor gekoppelt werden kann. Im Bereich des Förderelements 73 ist die Antriebswelle 71 mit einer Seele 75 verstärkt, wobei sich die Seele zwischen dem distalen Ende 72 und einem Bereich proximal des Förderelements 73 erstreckt. Das Förderelement 73 umfasst zwei flexible Segmente 76 und 77, welche bei einer Drehrichtung des Förderelements, vom proximalen zum distalen Ende betrachtet, im Uhrzeigersinn, eine Förderung eines Fluids vom distalen zum proximalen Ende hin bewirken. In der FIG. 6b ist ein Querschnitt des Rotors 73 vom proximalen Ende zum distalen Ende hin dargestellt. Hier ist die Form der flexiblen Segmente 76 und 77 detaillierter erkennbar. Die Faltrichtung des Rotors bei einem Einziehen des nicht dargestellten Pumpengehäuses in die Kanüle ist im Uhrzeigersinn, das heißt die Punkte 78 bzw. 79 werden radial nach innen und im Uhrzeigersinn transportiert. Entsprechend entfaltet sich der Rotor beim Herausschieben des Förderelements aus dem Katheter entgegen dem Uhrzeigersinn. Von daher ist es in einer Variante vorgesehen, dass dargestellte Förderelement bzw. die Wellenanordnung 70 mit einem Gehäuse zu versehen, welches derart ausgebildet ist, dass dieses ein Drehmoment im Uhrzeigersinn beim Überführen des Pumpengehäuses vom expandierten in den komprimierten Zustand hin bewirkt.

Die Seele 75 kann dabei eine verbesserte Steifigkeit gegenüber den übrigen Bereichen der hohlen Antriebswelle 71 bewirken. Die Seele kann dabei von ihrem distalen zu ihrem proximalen Ende hin unterschiedliche Steifigkeiten aufweisen, so dass beispielsweise die Biegesteifigkeit proximal und/oder distal des Förderelements gegenüber der Steifigkeit der Seele im Bereich des Förderelements verringert ist. Die entsprechende Steifigkeit der Welle im Bereich des Förderelements kann aber auch durch eine entsprechende Gestaltung (bzw. Abstimmung) der Rotornabe erreicht werden.

Anhand der FIG. 7a bis 7c sollen weitere Details eines Pumpengehäuses erläutert werden. In der FIG. 7a ist das in der FIG. 7b dargestellte Pumpengehäuse entlang einer fiktiven Trennlinie aufgetrennt, aufgerollt und flachgedrückt worden. In einer Ausführungsform wird allerdings zuerst das Pumpengehäuse, wie in der Fig. 7a dargestellt, beispielsweise mittels eines Lasers ausgeschnitten. Das Ausschneiden kann dabei innerhalb einer Rohrform durchgeführt werden. Anschließend wird die in der Fig. 7b dargestellte Form mittels eines Glühprozesses in einer Form durchgeführt. Das in der FIG. 7a ebenfalls entrollte Pumpengehäuse 80 weist an seinem proximalen Ende 81 den proximalen Endabschnitt 83 auf, welcher sich bis zu den spiralförmigen Elementen 82 hin erstreckt. Ein kurzer Bereich vor und nach den spiralförmigen Streben 82 definiert hierbei den proximalen Abschnitt 84. Der pumpenaufnehmende Abschnitt 85 weist ein Gittermuster auf, in welchem die gitterförmig miteinander verbundenen Streben Kontaktpunkte miteinander haben. Analog zum proximalen Abschnitt 84 zeigt der distale Abschnitt 86 spiralförmige Streben 87, welche in ihrer Spiralrichtung den Streben 82 entgegengerichtet sind. Am distalen Ende befindet sich ein distaler Endabschnitt 88, in dessen Bereich beispielsweise die Antriebswelle in einem Katheter oder Pigtail gelagert sein kann. Der Winkel, unter welchem die spiralförmigen Elemente 82 vom proximalen Endabschnitt zum pumpenaufnehmenden Abschnitt verlaufen, kann beispielsweise zwischen 20 und 40° betragen. Analog hierzu kann der Winkel der Streben 87 ebenfalls 20 bis 40° (allerdings in entgegen gesetzter Richtung) betragen.

In ihre Ausführungsform sind die beiden Winkel in ihrer Richtung entgegengesetzt, wie in der FIG. 7 dargestellt. Wird das Pumpengehäuse 80 nun, wie oben angedeutet, zusammengefügt, entsteht das Pumpengehäuse im expandierten Zustand wie in der FIG. 7b dargestellt. Es ist deutlich erkennbar, dass im Bereich des proximalen bzw. distalen Abschnitts 84 bzw. 86 eine Aufweitung des Innendurchmessers vom proximalen zum distalen Ende bzw. umgekehrt hin stattfindet. Der pumpenaufnehmende Abschnitt 85 weist dabei den größten Innendurchmesser auf, um eine hohe Effizienz bei der Förderung des Fluids zu erzielen. Ein Querschnitt des Pumpengehäuses 80, vom proximalen Ende zum distalen Ende betrachtet, ist in der FIG. 7c gezeigt, wobei deutlich erkennbar ist, dass die Streben 82 gegen den Uhrzeigersinn laufen. Weiterhin sind die Stützstreben 89 dargestellt, welche eine Überleitung zu den Gitterstreben 85a des pumpenaufnehmenden Abschnitts zeigen.

Anhand der FIG. 8 ist der distale Endabschnitt 88 des Pumpengehäuses 80 dargestellt. Ein Katheter 90 ist dabei in den distalen Endabschnitt 88 eingeschoben und umfasst unter anderem eine Lagerhülse 91, in welcher das distale Ende der Wellenanordnung 70 gelagert ist. Das Lager kann hierbei beispielsweise aus einer Keramik bestehen, wohingegen die Welle aus den vorab beschriebenen Werkstoffen aufgebaut sein kann.

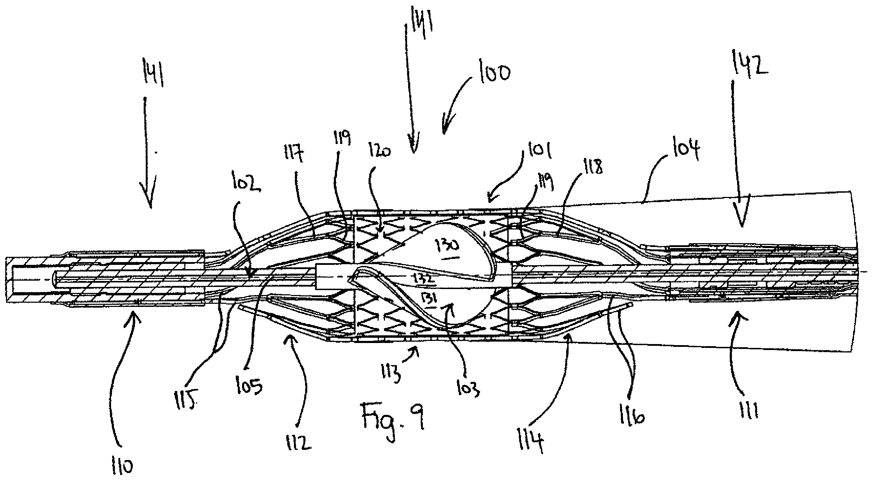

In der FIG. 9 ist Längsschnitt durch eine Pumpenanordnung 100 gezeigt, welche ein Pumpengehäuse 101, eine Antriebswelle 102 und einen auf der Antriebswelle angeordneten Rotor 103 umfasst. Weiterhin ist ein Abströmschlauch 104 dargestellt. Im distalen Endbereich 110 des Pumpengehäuses ist dieses mit einem Katheter verbunden, welcher als Pigtail ausgebildet ist (nicht dargestellt). Die Lagerung der Antriebswelle 102 im distalen Endabschnitt entspricht dabei im Wesentlichen der anhand der FIG. 8 erläuterten Lagerung.

Im Bereich des proximalen Endabschnitts 111 befindet sich eine proximale Lagerung 112 der Antriebswelle, welche sowohl ein Radiallager als auch ein Axiallager umfasst. Dieses Lager ist in der taggleich eingereichten Anmeldung mit dem internen Aktenzeichen 137EP 2457 genauer erläutert.

Zwischen den distalen und proximalen Endabschnitten des Pumpengehäuses 101 befinden sich der distale Abschnitt 112, der pumpenaufnehmenden Abschnitt 113 und der proximale Abschnitt 114. Dabei weisen sowohl der distale als auch der proximale Abschnitt spiralförmige Streben 115 bzw. 116 auf, welche zum pumpenaufnehmenden Abschnitt hin in Stützstreben 117 bzw. 118 übergehen. Diese Stützstreben spalten sich jeweils weiter in Streben 119 des pumpenaufnehmenden Abschnitts auf. An der Innenseite des pumpenaufnehmenden Abschnitts befindet sich ein Kunststofffilm 120, welcher in einem Ausführungsbeispiel aus einem Polyurethan hergestellt ist. Dieser Film verbessert die Förderwirkung des Rotors 103.

Der Rotor 103 umfasst zwei flexible Rotorblätter 130 und 131, welche an einer Nabe 132 befestigt sind. In einigen Ausführungsbeispielen ist der Rotor ein einziges Werkstück aus einem Kunststoff, wie beispielsweise Polyurethan, wie zum Beispiel Biresin oder aus einem Silikon oder auch Pebax. Aus Gründen der Übersichtlichkeit ist der Rotor 103 nicht in einer Längsschnittdarstellung gezeigt.

Der Rotor 103 ist auf einer Antriebswelle 102 angeordnet, welche als Hohlwelle ausgebildet ist. Hinsichtlich der Details wird auf die taggleich eingereichte Anmeldung PMP Ref. 137EP 2457 verwiesen. Zwischen der distalen und proximalen Lagerung ist die Hohlwelle mit einer Seele 105 verstärkt.

Bei der Abstimmung der Biegelinie des Pumpengehäuses auf die Biegelinie der Antriebswelle wird darauf geachtet, dass im Falle eines auf das Pumpengehäuse einwirkenden Biegemoments 140 (oder 141 oder 142), der Rotor 103 im Wesentlichen konzentrisch im pumpenaufnehmenden Abschnitt 113 verbleibt, bzw. das der Rotor nicht an die Innenfläche des pumpenaufnehmenden Abschnitts 113 anstößt. Als erste Maßnahme ist in diesem Ausführungsbeispiel die Biegesteifigkeit des pumpenaufnehmenden Abschnitts steifer als die Biegesteifigkeit des distalen bzw. proximalen Abschnitts. Im gezeigten Ausführungsbeispiel sind der Einfachheit halber die Biegesteifigkeiten des distalen und proximalen Abschnitts symmetrisch gewählt. Eine Möglichkeit die Biegesteifigkeit im pumpenaufnehmenden Abschnitt 113 zu beeinflussen ist die Dichte und Anzahl der Streben 119 bezogen auf den betrachteten Durchmesser des Gehäuses. Im vorliegenden Beispiel weisen der distale und proximale Abschnitt 112 bzw. 114 jeweils 10 spiralförmige Streben auf, welche zum pumpenaufnehmenden Abschnitt hin in jeweils 20 Stützstreben 117 bzw. 118 übergehen. Die Stützstreben 117 bzw. 118 spalten sich nochmals in 40 Streben 119 auf, so dass die Anzahl der Streben im pumpenaufnehmenden Bereich hier um einen Faktor 4 höher ist. In anderen Ausführungsbeispielen kann dieser Faktor zwischen 0,9 und 20 variieren. Auf diese Weise ist die Biegesteifigkeit im pumpenaufnehmenden Abschnitt höher als im distalen oder proximalen Bereich.

Eine weitere Möglichkeit die Biegesteifigkeiten des distalen und proximalen Abschnitts gegenüber dem pumpenaufnehmenden Abschnitt anzupassen (hier: weicher zu machen) ist die Änderung der geometrischen Abmaße der Streben 115-119. Im vorliegenden Beispiel sind die Streben 115 bzw. 116 im einen Faktor 2 bis 3 dicker als die Streben 119. Aufgrund des Faktors 4 beim Verhältnis der Anzahl der Streben wären der proximale und distale Abschnitt sonst in einigen Ausführungsbeispielen zu weich, sofern die Streben 115-119 gleichdick wären.

Eine weitere Möglichkeit die Biegesteifigkeit im Bereich der proximalen und distalen Abschnitte anzupassen ist die Wahl des Biegewinkels der spiralförmigen Streben. Im vorliegenden Beispiel winden sich die spiralförmigen Streben um einen Winkel von ca. 30° vom distalen zum proximalen Ende des proximalen bzw. distalen Abschnitts hin. Die Bereich kann jedoch auch in einem Bereich von 5° bis 90° liegen.

Eine weitere Möglichkeit ist es die Länge des proximalen und distalen Abschnitts zu variieren. In einem Verfahren zur Abstimmung der Biegesteifigkeit des Pumpengehäuses wird zunächst die Wellenanordnung vermessen und anschließend die oben genannten Parameter der verschiedenen Abschnitte des Pumpengehäuses berechnet und anschließend ein geeignetes Pumpengehäuse hergestellt.

Die Biegesteifigkeit der Antriebswelle kann durch die Steifigkeit der Hohlwelle, der Seele und des Rotors angepasst werden. Da die Hohlwelle in einigen Ausführungsbeispielen starken Krümmungen, z.B. im Aortenbogen, ausgesetzt sein kann, muss die Hohlwelle eine Biegesteifigkeit besitzen, welche eine derartige Krümmung erlaubt und zugleich eine Festigkeit besitzt um möglichst lange unter hohen Drehzahlen operieren zu können. Von daher bietet es sich in einigen Ausführungsbeispielen an die Biegesteifigkeit der Hohlwelle primär an die Anforderungen an die Hohlwelle zwischen Motor und Lagerung anzupassen. Die Steifigkeit der Seele kann jedoch angepasst werden, um die Biegelinie der Antriebswelle an die Biegesteifigkeit des Pumpengehäuses zwischen dem proximalen und distalen Lager anzupassen.

Des Weiteren bewirkt die Materialwahl und Geometrie des Rotors 103 eine Versteifung der Antriebswelle im Bereich des pumpenaufnehmenden Abschnitts 113, so dass die Antriebswellenanordnung mit Rotor im Bereich des distalen und proximalen Abschnitts weicher ist als im Bereich des pumpenaufnehmenden Abschnitts. Weitere Anpassungsmöglichkeiten ergeben sich für den Fachmann aus den hier gemachten Ausführungen.

In einem weiteren Ausführungsbeispiel weist das Pumpengehäuse eine spiralförmige Struktur auf, welche als Ergebnis einer Vielzahl von miteinander verbundenen Streben, auftritt. Durch die Wahl des Verbindungspunkts zweier Streben kann, obgleich die Streben sich schräg nach Oben bzw. Unten erstrecken, eine spiralförmige, in eine Richtung gerichtete Struktur entstehen. Die Biegesteifigkeit dieser Struktur kann durch Änderungen der Dicke, Anzahl, Länge und dem umfassten Winkel der Struktur an die Biegesteifigkeit der Antriebswelle angeordnet werden.

Weitere Ausführungsformen und Varianten der Erfindung ergeben sich aus den hier genannten Kombinationen und den Kombinationen, welche für den Fachmann ersichtlich sind.

Die Erfindung wird durch die Ansprüche definiert. Teil der Offenbarung bilden zudem die folgenden Aspekte, die zur Erläuterung der Erfindung dienen können:
Pumpe, insbesondere Blutpumpe (1), mit einem proximalen und einem distalen Ende und einem dazwischen angeordneten Pumpengehäuse (2; 20; 40; 80), einer in einem Innenraum des Pumpengehäuses entlang einer Längsrichtung angeordneten Antriebswelle (4; 21; 48;71), einem auf der Antriebswelle angeordnetem Förderelement (5; 22; 49; 73) sowie einer Kanüle (3;30; 47),
wobei das Pumpengehäuse derart ausgebildet ist, dass das Pumpengehäuse unter Beaufschlagung mit einer zum proximalen Ende der Pumpe hin wirkenden Kraft zumindest teilweise in die Kanüle überführbar ist, und dabei zumindest entlang einer quer zur Längsrichtung verlaufenden Radialrichtung von einem expandierbaren Zustand in einen komprimierten Zustand überführt wird; und wobei das Pumpengehäuse weiterhin mindestens einen Abschnitt aufweist, welcher im expandierten Zustand entlang der Längsachse spiralförmig um die Längsachse verlaufende Strukturen (27; 46, 50; 82, 87) umfasst, welche vom proximalen zum distalen Ende der Pumpe betrachtet in einer ersten Richtung gewunden sind;
und wobei das Förderelement mindestens ein faltbares Segment (22, 23; 54, 55; 76, 77) umfasst,
wobei
die spiralförmig verlaufenden Strukturen derart ausgebildet sind, dass beim Überführen des Pumpengehäuses vom expandierten in den komprimierten Zustand ein entgegen der ersten Richtung gerichtetes Drehmoment (31; 51) auf das faltbare Segment wirkt.
2. Pumpe nach Aspekt 1, wobei das faltbare Segment des Förderelements derart ausgebildet ist, dass das Drehmoment einer Drehrichtung (34; 59) des Förderelements zur Förderung eines Fluids vom distalen zum proximalen Ende der Pumpe hin gleichgerichtet ist.
3. Pumpe nach Aspekt 1, wobei das faltbare Segment des Förderelements derart ausgebildet ist, dass das Drehmoment einer Förderrichtung des Förderelements zur Förderung eines Fluids vom distalen zum proximalen Ende der Pumpe hin entgegengesetzt ist.
4. Pumpe nach einem der vorhergehenden Aspekte, wobei eine Entfaltrichtung (33; 57) des mindestens einen faltbaren Segments beim Entfalten in der ersten Richtung verläuft.
5. Pumpe nach einem der Aspekte 1 bis 3, wobei eine Entfaltrichtung des mindestens einen faltbaren Segments beim Entfalten in einer der ersten Richtung entgegengesetzten Richtung verläuft.
6. Pumpe nach einem der vorhergehenden Aspekte, wobei das Pumpengehäuse aus einem Memory-Shape Material hergestellt ist.
7. Pumpe nach einem der vorhergehenden Aspekte, wobei das Pumpengehäuse einen pumpenaufnehmenden (41; 85) und einen proximal des pumpenaufnehmenden Abschnitts angeordneten proximalen Abschnitt (44; 84) umfasst, wobei ein Innendurchmesser des proximalen Abschnitts von einem Durchmesser des pumpenaufnehmenden Abschnitts im expandierten Zustand des Pumpengehäuses zu einem proximalen Ende des proximalen Abschnitts hin verringert.
8. Pumpe nach Aspekt 7, wobei die spiralförmigen Streben im proximalen Abschnitt angeordnet sind.
9. Pumpe nach Aspekt 7, wobei die spiralförmigen Streben im pumpenaufnehmenden Abschnitt angeordnet sind.
10. Pumpe nach einem der Aspekte 7 bis 9, wobei das Pumpengehäuse einen weiteren, distal des pumpenaufnehmenden Abschnitts angeordneten distalen Abschnitt ( 42; 86) umfasst, dessen Innendurchmesser vorzugsweise von einem Durchmesser des pumpenaufnehmenden Abschnitts im expandierten Zustand des Pumpengehäuses zu einem distalen Ende des distalen Abschnitts hin verringert.
11. Pumpe nach Aspekt 10, wobei die spiralförmigen Streben im distalen Abschnitt angeordnet sind.
12. Pumpe nach einem der vorhergehenden Aspekte, wobei die Antriebswelle zumindest in einem Bereich eines proximalen Endes (45; 83) des Pumpengehäuses gelagert ist.
13. Pumpe nach Aspekt 12, wobei die Antriebswelle zusätzlich in einem Bereich eines distalen Endes (43; 87) des Pumpengehäuses gelagert ist.
14. Pumpe nach einem der vorhergehenden Aspekte, wobei die spiralförmigen Strukturen spiralförmige Streben umfassen.
15. Pumpe, insbesondere Blutpumpe, mit einem proximalen und einem distalen Ende und einem dazwischen angeordneten Pumpengehäuse, einer in einem Innenraum des Pumpengehäuses entlang einer Längsrichtung angeordneten Antriebswelle, einem auf der Antriebswelle angeordnetem Förderelement,
   wobei das Förderelement mindestens ein flexibles Segment umfasst, welches derart ausgebildet ist, dass eine Drehrichtung des Förderelements eine Förderung eines Fluids vom distalen zum proximalen Ende der Pumpe hin bewirkt;
   und das Pumpengehäuse derart ausgebildet ist, dass das Pumpengehäuse unter Beaufschlagung mit einer zum proximalen Ende der Pumpe hin wirkenden Kraft zumindest teilweise in eine Kanüle überführbar ist, und dabei zumindest entlang einer quer zur Längsrichtung verlaufenden Radialrichtung von einem expandierbaren Zustand in einen komprimierten Zustand überführt wird;
   wobei
   eine Entfaltrichtung des mindestens einen flexiblen Segments bei einem Überführen des Pumpengehäuses vom komprimierten in den expandierten Zustand entgegen der Drehrichtung gerichtet ist.
16. Pumpe, insbesondere Blutpumpe, mit einem Pumpengehäuse, einer in einem Innenraum des Pumpengehäuses entlang einer Längsachse angeordneten Antriebswelle sowie einem auf der Antriebswelle angeordneten Förderelement,
   wobei das Pumpengehäuse
      mindestens einen pumpenaufnehmenden Abschnitt und einen proximal des pumpenaufnehmenden Abschnitts angeordneten proximalen Abschnitts umfasst, und in einer quer zur Längsrichtung verlaufenden Radialrichtung von einem komprimierten Zustand in einen expandierten Zustand überführbar ist;
   und wobei die Antriebswelle im Bereich des proximalen Endes des Pumpengehäuses in einem proximalen Lager gelagert ist,
   wobei
   die Antriebswelle derart konfiguriert ist, dass eine Biegesteifigkeit der Antriebswelle im Bereich des proximalen Abschnitts des Pumpengehäuses und distal des proximalen Lagers auf eine Biegesteifigkeit des proximalen Abschnitts abgestimmt ist, so dass bei einer Biegung des Pumpengehäuses das Förderelement im Wesentlichen konzentrisch innerhalb des pumpenaufnehmenden Abschnitts angeordnet ist.
17. Pumpe nach Aspekt 16, wobei das Pumpengehäuse weiterhin einen distal des pumpenaufnehmenden Abschnitts angeordneten distalen Abschnitt umfasst, wobei die Antriebswelle im Bereich des distalen Endes des Pumpengehäuses in einem distalen Lager gelagert ist, und eine Biegesteifigkeit der Antriebswelle im Bereich des distalen Abschnitts und proximal des distalen Abschnitts derart auf eine Biegesteifigkeit des distalen Abschnitts abgestimmt ist, so dass bei einer Biegung des Pumpengehäuses das Förderelement im Wesentlichen konzentrisch innerhalb des pumpenaufnehmenden Abschnitts angeordnet ist.
18. Pumpe nach Aspekt 16, wobei die Biegesteifigkeit des proximalen Abschnitts im Wesentlichen durch die Biegesteifigkeit entlang der Längsachse verlaufender spiralförmiger Streben bestimmt ist und/oder das Pumpengehäuse weiterhin einen distal des pumpenaufnehmenden Abschnitts angeordneten distalen Abschnitt umfasst, wobei die Antriebswelle im Bereich des distalen Endes des Pumpengehäuses in einem distalen Lager gelagert ist, und eine Biegesteifigkeit der Antriebswelle im Bereich des distalen Abschnitts und proximal des distalen Abschnitts derart auf eine Biegesteifigkeit des distalen Abschnitts abgestimmt ist, so dass bei einer Biegung des Pumpengehäuses das Förderelement im Wesentlichen konzentrisch innerhalb des pumpenaufnehmenden Abschnitts angeordnet ist und die Biegesteifigkeit des distalen Abschnitts im Wesentlichen durch die Biegesteifigkeit entlang der Längsachse verlaufender spiralförmiger Streben bestimmt ist.
19. Pumpe nach einem der Aspekte 16 bis 18, wobei die Antriebswelle eine Hohlwelle ist, welche im Bereich des Pumpengehäuses eine Seele umfasst.
20. Pumpe nach einem der vorhergehenden Aspekte, wobei eine "austentite finish" (A_{f})-Temperatur des Pumpengehäuses unterhalb einer Temperatur von 34°C, vorzugsweise unterhalb von 30°C, besonders vorzugsweise unter 20°C liegt.

## Patentansprüche

1. Pumpe, insbesondere Blutpumpe (1), mit einem proximalen und einem distalen Ende und einem dazwischen angeordneten Pumpengehäuse (2, 20, 40, 80), einer in einem Innenraum des Pumpengehäuses (2, 20, 40, 80) entlang einer Längsrichtung angeordneten Antriebswelle (4, 21, 48, 71), einem auf der Antriebswelle (4, 21, 48, 71) angeordnetem Förderelement (5, 22, 49, 73),
wobei das Förderelement (5, 22, 49, 73) mindestens ein flexibles Segment umfasst, welches derart ausgebildet ist, dass eine Drehrichtung des Förderelements (5, 22, 49, 73) eine Förderung eines Fluids vom distalen zum proximalen Ende der Pumpe hin bewirkt;
und das Pumpengehäuse (2, 20, 40, 80) derart ausgebildet ist, dass das Pumpengehäuse (2, 20, 40, 80) unter Beaufschlagung mit einer zum proximalen Ende der Pumpe hin wirkenden Kraft zumindest teilweise in eine Kanüle (3, 30, 47) überführbar ist, und dabei zumindest entlang einer quer zur Längsrichtung verlaufenden Radialrichtung von einem expandierbaren Zustand in einen komprimierten Zustand überführt wird;
wobei
eine Entfaltrichtung des mindestens einen flexiblen Segments bei einem Überführen des Pumpengehäuses (2, 20, 40, 80) vom komprimierten in den expandierten Zustand entgegen der Drehrichtung gerichtet ist,
wobei das Pumpengehäuse (2, 20, 40, 80) einen pumpenaufnehmenden (41; 85) und einen proximal des pumpenaufnehmenden Abschnitts angeordneten proximalen Abschnitt (44; 84) umfasst, wobei ein Innendurchmesser des proximalen Abschnitts von einem Durchmesser des pumpenaufnehmenden Abschnitts im expandierten Zustand des Pumpengehäuses (2, 20, 40, 80) zu einem proximalen Ende des proximalen Abschnitts hin verringert,
wobei das Pumpengehäuse (2, 20, 40, 80) weiterhin mindestens einen Abschnitt aufweist, welcher im expandierten Zustand entlang der Längsachse spiralförmig um die Längsachse verlaufende Strukturen (27; 46, 50; 82, 87) umfasst, welche vom proximalen zum distalen Ende der Pumpe betrachtet in einer ersten Richtung gewunden sind,
wobei die spiralförmigen Strukturen (27; 46, 50; 82, 87) spiralförmige Streben umfassen,
wobei die spiralförmigen Streben im proximalen Abschnitt (44; 84) angeordnet sind.

2. Pumpe nach Anspruch 1, wobei das Pumpengehäuse (2, 20, 40, 80) aus einem Memory-Shape Material hergestellt ist.

3. Pumpe nach Anspruch 1 oder 2, wobei eine "austenite finish" (A_{f})-Temperatur des Pumpengehäuses (2, 20, 40, 80) unterhalb einer Temperatur von 34°C, vorzugsweise unterhalb von 30°C, besonders vorzugsweise unter 20°C liegt.

4. Pumpe nach einem der Ansprüche 1 bis 3, wobei die spiralförmig verlaufenden Strukturen derart ausgebildet sind, dass beim Überführen des Pumpengehäuses (2, 20, 40, 80) vom expandierten in den komprimierten Zustand ein entgegen der ersten Richtung gerichtetes Drehmoment (31; 51) auf das faltbare Segment wirkt.

5. Pumpe nach einem der Ansprüche 1 bis 4, wobei die spiralförmigen Streben im pumpenaufnehmenden Abschnitt angeordnet sind.

6. Pumpe nach einem der Ansprüche 1 bis 5, wobei das Pumpengehäuse (2, 20, 40, 80) einen weiteren, distal des pumpenaufnehmenden Abschnitts angeordneten distalen Abschnitt (42; 86) umfasst, dessen Innendurchmesser vorzugsweise von einem Durchmesser des pumpenaufnehmenden Abschnitts im expandierten Zustand des Pumpengehäuses (2, 20, 40, 80) zu einem distalen Ende des distalen Abschnitts hin verringert.

7. Pumpe nach Anspruch 6, wobei die spiralförmigen Streben im distalen Abschnitt angeordnet sind.

8. Pumpe nach einem der vorhergehenden Ansprüche, wobei die Antriebswelle (4, 21, 48, 71) zumindest in einem Bereich eines proximalen Endes (45; 83) des Pumpengehäuses (2, 20, 40, 80) gelagert ist.

9. Pumpe nach Anspruch 8, wobei die Antriebswelle (4, 21, 48, 71) zusätzlich in einem Bereich eines distalen Endes (43; 87) des Pumpengehäuses (2, 20, 40, 80) gelagert ist.

10. Pumpe nach einem der vorhergehenden Ansprüche, wobei die Antriebswelle (4, 21, 48, 71) eine Hohlwelle ist, welche im Bereich des Pumpengehäuses (2, 20, 40, 80) eine Seele umfasst.

## Claims

1. A pump, in particular a blood pump (1), with a proximal and a distal end and a pump housing (2, 20, 40, 80) arranged therebetween, a drive shaft (4, 21, 48, 71) arranged in an interior space of the pump housing (2, 20, 40, 80) along a longitudinal direction, a conveying element (5, 22, 49, 73) arranged on the drive shaft (4, 21, 48, 71),
wherein the conveying element (5, 22, 49, 73) comprises at least one flexible segment configured such that a direction of rotation of the conveying element (5, 22, 49, 73) causes a fluid to be conveyed from the distal end to the proximal end of the pump;
and the pump housing (2, 20, 40, 80) is configured such that the pump housing (2, 20, 40, 80) is at least partially transferable into a cannula (3, 30, 47) under action of a force acting towards the proximal end of the pump, and is thereby transitioned from an expandable state into a compressed state at least along a radial direction extending transversely to the longitudinal direction;
wherein
an unfolding direction of the at least one flexible segment during a transitioning of the pump housing (2, 20, 40, 80) from the compressed to the expanded state is directed opposite to the direction of rotation,
wherein the pump housing (2, 20, 40, 80) comprises a pump receiving portion (41; 85) and a proximal portion (44; 84) disposed proximally of the pump receiving portion, wherein an inner diameter of the proximal portion decreases from a diameter of the pump receiving portion in the expanded state of the pump housing (2, 20, 40, 80) towards a proximal end of the proximal portion,
wherein the pump housing (2, 20, 40, 80) further comprises at least one portion which, in the expanded state, comprises structures (27; 46, 50; 82, 87) extending along the longitudinal axis spirally about the longitudinal axis, the structures being wound in a first direction as viewed from the proximal to the distal end of the pump,
wherein the spiral structures (27; 46, 50; 82, 87) comprise spiral struts,
wherein the spiral struts are disposed in the proximal portion (44; 84).

2. The pump according to claim 1, wherein the pump housing (2, 20, 40, 80) is made of a memory shape material.

3. The pump according to any one of claims 1 or 2, wherein an "austenite finish" (A_{f}) temperature of the pump housing (2, 20, 40, 80) is below a temperature of 34°C, preferably below 30°C, particularly preferably below 20°C.

4. The pump according to any one of claims 1 to 3, wherein the spirally extending structures are configured such that when the pump housing (2, 20, 40, 80) is transferred from the expanded to the compressed state, a torque (31; 51) directed against the first direction acts on the foldable segment.

5. The pump according to any one of claims 1 to 4, wherein the spiral struts are disposed in the pump receiving portion.

6. A pump according to any one of claims 1 to 5, wherein the pump housing (2, 20, 40, 80) comprises a further distal portion (42; 86) disposed distally of the pump receiving portion, the inner diameter of which preferably decreases from a diameter of the pump receiving portion in the expanded state of the pump housing (2, 20, 40, 80) towards a distal end of the distal portion.

7. The pump according to claim 6, wherein the spiral struts are disposed in the distal portion.

8. The pump according to any one of the preceding claims, wherein the drive shaft (4, 21, 48, 71) is supported in at least a portion of a proximal end (45; 83) of the pump housing (2, 20, 40, 80).

9. The pump of claim 8, wherein the drive shaft (4, 21, 48, 71) is additionally supported in a region of a distal end (43; 87) of the pump housing (2, 20, 40, 80).

10. The pump according to any of the preceding claims, wherein the drive shaft (4, 21, 48, 71) is a hollow shaft which comprises a core in the region of the pump housing (2, 20, 40, 80).

## Revendications

1. Pompe, en particulier pompe à sang (1), avec une extrémité proximale et une extrémité distale et un boîtier de pompe (2, 20, 40, 80) agencé entre elles, un arbre d'entraînement (4, 21, 48, 71) agencé dans un espace intérieur du boîtier de pompe (2, 20, 40, 80) le long d'une direction longitudinale, un élément de transport (5, 22, 49, 73) agencé sur l'arbre d'entraînement (4, 21, 48, 71),
dans laquelle l'élément de transport (5, 22, 49, 73) comprend au moins un segment flexible qui est conçu de telle sorte qu'une direction de rotation de l'élément de transport (5, 22, 49, 73) provoque un transport d'un fluide à partir de la partie distale vers l'extrémité proximale de la pompe ;
et le boîtier de pompe (2, 20, 40, 80) est conçu de telle sorte que le boîtier de pompe (2, 20, 40, 80) peut être transféré au moins partiellement dans une canule (3, 30, 47), par l'application d'une force agissant sur l'extrémité proximale de la pompe, et est ainsi transféré d'un état expansible à un état comprimé au moins le long d'une direction radiale s'étendant transversalement à la direction longitudinale ;
dans laquelle
une direction de déploiement du au moins un segment flexible lorsque le boîtier de pompe (2, 20, 40, 80) est transféré de l'état comprimé à l'état expansé est orientée à l'inverse de la direction de rotation,
dans laquelle le boîtier de pompe (2, 20, 40, 80) comprend une section de réception de pompe (41 ; 85) et une section proximale (44 ; 84) qui est proximale de la section de réception de pompe, dans laquelle un diamètre intérieur de la section proximale diminue à partir d'un diamètre de la section de réception de pompe à l'état expansé du boîtier de pompe (2, 20, 40, 80) vers une extrémité proximale de la section proximale,
dans laquelle le boîtier de pompe (2, 20, 40, 80) présente en outre au moins une section qui, à l'état expansé, comprend des structures (27 ; 46, 50 ; 82, 87) s'étendant de manière hélicoïdale autour de l'axe longitudinal le long de l'axe longitudinal qui, vues de l'extrémité proximale vers l'extrémité distale de la pompe, sont enroulées dans une première direction,
dans laquelle les structures hélicoïdales (27 ; 46, 50 ; 82, 87) comprennent des entretoises hélicoïdales,
dans laquelle les entretoises hélicoïdales sont agencées dans la section proximale (44 ; 84).

2. Pompe selon la revendication 1, dans laquelle le boîtier de pompe (2, 20, 40, 80) est fabriqué en un matériau à mémoire de forme.

3. Pompe selon la revendication 1 ou 2, dans laquelle une température de « finition austénitique » (A_{f}) du boîtier de pompe (2, 20, 40, 80) est inférieure à une température de 34°C, de préférence inférieure à 30°C, de manière particulièrement préférée inférieure à 20°C.

4. Pompe selon l'une quelconque des revendications 1 à 3, dans laquelle les structures s'étendant de manière hélicoïdale sont conçues de telle sorte que lorsque le boîtier de pompe (2, 20, 40, 80) est transféré de l'état expansé à l'état comprimé, un couple de rotation (31 ; 51) orienté à l'inverse de la première direction agit sur le segment pliable.

5. Pompe selon l'une quelconque des revendications 1 à 4, dans laquelle les entretoises hélicoïdales sont agencées dans la section de réception de pompe.

6. Pompe selon l'une quelconque des revendications 1 à 5, dans laquelle le boîtier de pompe (2, 20, 40, 80) comprend une section distale supplémentaire (42 ; 86) agencée de manière distale par rapport à la section de réception de pompe, dont le diamètre intérieur diminue de préférence d'un diamètre de la section de réception de pompe dans l'état expansé du boîtier de pompe (2, 20, 40, 80) vers une extrémité distale de la section distale.

7. Pompe selon la revendication 6, dans laquelle les entretoises hélicoïdales sont agencées dans la section distale.

8. Pompe selon l'une quelconque des revendications précédentes, dans laquelle l'arbre d'entraînement (4, 21, 48, 71) est monté au moins dans une zone d'une extrémité proximale (45 ; 83) du boîtier de pompe (2, 20, 40, 80).

9. Pompe selon la revendication 8, dans laquelle l'arbre d'entraînement (4, 21, 48, 71) est en outre monté dans une zone d'une extrémité distale (43 ; 87) du boîtier de pompe (2, 20, 40, 80).

10. Pompe selon l'une quelconque des revendications précédentes, dans laquelle l'arbre d'entraînement (4, 21, 48, 71) est un arbre creux qui comprend une âme au niveau du boîtier de pompe (2, 20, 40, 80).
